# FASCICULE DE BREVET EUROPEEN

(11) **EP 0 642 781 B1**
(45) Date de publication et mention de la délivrance du brevet: **20.01.1999**
(21) Numéro de dépôt: 94401898.5
(22) Date de dépôt: 25.08.1994
(51) Int. Cl.: A61K 7/06, A61K 7/00, A61K 7/48

(54) **Emulsions acides stables de type huile-dans-eau**
Stabile Säure Öl-in-Wasser Emulsionen
Stable acid oil-in-water emulsions

(30) Priorité: 15.09.1993 FR 9310966
(43) Date de publication de la demande: 15.03.1995
(73) Titulaire: L'OREAL, 75008 Paris (FR)
(72) Inventeur: Griat, Jacqueline, F-94480 Ablon (FR)
(74) Mandataire: Lhoste, Catherine

(56) Documents cités:
- EP-A- 0 503 853
- FR-A- 2 681 245

## Description

La présente invention a pour objet de nouvelles émulsions de type huile-dans-eau (H/E), c'est à dire des émulsions dans lesquelles la phase continue dispersante est aqueuse et la phase discontinue dispersée est huileuse, à la fois nettement acides et parfaitement stables, ainsi que des compositions cosmétiques et/ou dermatologiques et/ou pharmaceutiques les contenant.

Elle a également pour objet l'utilisation d'un polymère bien particulier dans le but de stabiliser des émulsions de type H/E, ou d'autres compositions susceptibles d'être à base de telles émulsions, présentant des pH nettement acides.

Pour diverses raisons liées en particulier à leur grand confort d'utilisation (douceur, émollience et autres), les émulsions de type huile-dans-eau sont aujourd'hui largement employées dans le domaine des compositions cosmétiques ou pharmaceutiques destinées à un usage topique (peau, cheveux).

Outre les classiques agents émulsionnants qui sont habituellement nécessaires à leur préparation, ces émulsions contiennent généralement, tant dans la phase aqueuse que dans la phase huileuse (encore appelée phase grasse), des principes cosmétiquement ou thérapeutiquement actifs qui sont appelés à être libérés lors de l'application sur le corps de la composition renfermant l'émulsion.

Certaines émulsions de type huile-dans-eau, bien que contenant des agents émulsionnants (ou tensio-actifs) comme indiqué ci-dessus, sont chimiquement peu stables, en particulier lorsque ces émulsions sont fortement concentrées en phase grasse et/ou lorsque celles-ci contiennent des agents non compatibles avec le reste des constituants formant l'émulsion. Ce manque, ou cette absence, de stabilité se traduit, dans les faits, par une séparation (par décantation) des phases aqueuse et huileuse de l'émulsion, cette séparation pouvant être plus ou moins progressive et/ou plus ou moins totale selon les cas.

Aussi, pour éviter ce phénomène indésirable, il est souvent nécessaire de faire appel à des agents dits épaississants, que l'on introduit alors dans l'émulsion et dont la fonction première est de créer, au sein de la phase aqueuse, une matrice gélatineuse servant à figer (ou "emprisonner") dans son réseau tridimensionnelle les particules (ou globules) de la phase grasse, assurant ainsi une sorte de stabilisation ou maintien mécanique de l'ensemble de l'émulsion. Cette manière d'opérer, bien qu'efficace, modifie toutefois de manière substantielle les propriétés rhéologiques de l'émulsion initiale, ce qui n'est pas toujours compatible avec certaines des applications.envisagées pour la composition finale.

La technique ci-dessus de stabilisation n'est malheureusement pas d'application générale, c'est à dire qu'elle ne convient pas pour toutes les émulsions de type huile-dans-eau pour lesquelles une stabilisation serait nécessaire ou souhaitable. Tel est ainsi le cas des émulsions qui présentent des pH nettement acides, en particulier des pH inférieurs ou égaux à 3,5, et encore plus particulièrement inférieurs ou égaux à 3, et qui, intrinsèquement, sont chimiquement peu stables. De telles émulsions acides peuvent se rencontrer notamment lorsque l'on cherche à obtenir des compositions cosmétiques et/ou pharmaceutiques contenant des principes actifs acides, comme par exemple des hydroxyacides à propriétés exfoliantes/hydratantes, compositions acides pour lesquelles il existe actuellement un fort besoin dans l'état de l'art.

En effet, lorsque l'on cherche à stabiliser de manière classique de telles émulsions acides (i.e de pH inférieurs ou égaux à 3,5) au moyen des agents épaississants usuels, on constate non seulement que ces derniers n'y exercent pas leur fonction épaississante première connue, mais également qu'ils produisent un effet de déstabilisation et de destruction contraire à l'effet recherché, conduisant à une séparation des phases aqueuse et huileuse de l'émulsion initiale. A la connaissance de la Demanderesse, il n'existe pas à ce jour de méthode simple, efficace et fiable, permettant de préparer et d'obtenir des émulsions acides et stables de type huile-dans-eau.

La présente invention a notamment pour but de résoudre les problèmes ci-dessus.

Plus précisément encore, la présente invention vise à mettre à disposition des émulsions de type huile-dans-eau qui soient acides, c'est à dire ici présentant un pH inférieur ou égal à 3,5, et encore plus particulièrement inférieur ou égal à 3, tout en ayant un caractère parfaitement stable. Elle vise également à mettre à disposition diverses compositions à base de telles émulsions et présentant les mêmes propriétés.

Ainsi, à la suite d'importantes recherches menées sur la question, il a été trouvé par la Demanderesse, et ceci de façon inattendue et surprenante, qu'en mettant en oeuvre un polymère bien particulier et seul convenable dans le cas présent, que l'on introduit dans une émulsion de type huile-dans-eau présentant initialement un pH inférieur ou égal à 3,5 et un état non stable, il est possible d'obtenir une émulsion finale également de type huile-dans-eau, de même pH, mais cette fois parfaitement stable.

Cette découverte est à la base de la présente invention.

Conformément à l'un des objets de la présente invention, il est donc maintenant proposé de nouvelles émulsions stables de type huile-dans-eau et de pH inférieur ou égal à 3,5, en particulier de pH inférieur ou égal à 3, lesdites émulsions comprenant, à titre d'agent stabilisant, un copolymère anionique réticulé substantiellement soluble dans l'eau et constitué de motifs dérivant de la réaction entre (i) l'acrylamide (monomère 1), (ii) l'acide 2-acrylamido 2-méthyl propane sulfonique (monomère 2, ci-après dénommé par commodité AMPS) et (iii) au moins un composé à polyinsaturation oléfinique (monomère 3, constituant ici l'agent de réticulation), ledit composé à polyinsaturation oléfinique étant présent dans le copolymère à une concentration comprise entre 0,06 et 1 millimole par mole de l'ensemble des motifs monomères.

Un autre aspect de la présente invention est constituée par l'utilisation d'un polymère tel que ci-dessus défini pour stabiliser des émulsions de type huile-dans-eau présentant un pH inférieur ou égal à 3,5, en particulier inférieur ou égal à 3, ainsi que par le procédé de stabilisation en découlant. Selon l'invention, la méthode se ramène simplement à introduire le polymère dans l'émulsion acide initiale à stabiliser.

On notera d'ores et déjà que la présente invention est de portée très générale, c'est à dire qu'elle convient à la stabilisation de toute émulsion acide (de pH inférieur ou égal à 3,5) de type huile-dans-eau, déjà connue en soi ou non, la composition de cette dernière ne revêtant en fait aucun caractère critique.

Un autre aspect enfin de la présente invention est constitué par les diverses compositions tant cosmétiques que pharmaceutiques susceptibles d'être préparées et obtenues à partir des émulsions conformes à l'invention. A cet égard, l'invention convient particulièrement bien à la préparation et à l'obtention de compositions se présentant sous la forme d'émulsions de type ci-dessus et contenant des principes actifs, cosmétiques et/ou thérapeutiques, à caractère nettement acide.

Mais d'autres caractéristiques, aspects et avantages de l'invention apparaîtront encore plus clairement à la lecture de la description détaillée qui va suivre, ainsi que des divers exemples destinés à l'illustrer de manière concrête mais nullement limitative.

Les copolymères anioniques réticulés utilisés à titre d'agents stabilisants dans le cadre de la présente invention sont des produits déjà connus en soi et leur préparation a été décrite notamment dans la demande de brevet EP-A-0 503 853. Il convient de noter ici que le document susmentionné ne concerne absolument pas, ni ne décrit, ni n'apporte d'enseignement utile sur, la résolution du problème bien particulier de la stabilisation des émulsions fortement acides de type huile-dans-eau.

Les copolymères ci-dessus peuvent ainsi être obtenus classiquement selon la technique dite de polymérisation en émulsion à partir des trois différents comonomères rentrant dans leur constitution.

Les monomères à polyinsaturation oléfinique utilisés comme agents de réticulation pour la préparation des copolymères conformes à l'invention sont de préférence choisis dans le groupe constitué par le méthylène bis-acrylamide, l'allyle-sucrose et le pentaérythritol. Encore plus préférentiellement, on fait appel au méthylène bis-acrylamide.

Le rapport, exprimé en moles %, entre l'acrylamide et l'AMPS est généralement compris entre 85/15 et 15/85, de préférence entre 70/30 et 30/70, encore plus préférentiellement entre 65/35 et 35/65, et plus particulièrement encore entre 60/40 et 40/60. En outre, l'AMPS est généralement au moins partiellement neutralisé sous la forme d'un sel, par exemple par de la soude, par de la potasse, ou par une amine à faible poids moléculaire telle que la triéthanolamine, ou leurs mélanges.

Un copolymère réticulé particulièrement préféré dans le cadre de la mise en oeuvre de la présente invention correspond à celui tel que préparé à l'exemple 1 de la demande de brevet EP-A- 0 503 853 précitée, et qui se présente alors sous la forme d'une émulsion inverse eau-dans-huile. Plus précisément, ce copolymère est constitué de 60% en moles d'acrylamide et de 40% en moles de sel de sodium d'AMPS, et il est réticulé par du méthylène bis-acrylamide à raison de 0,22 millimole par mole du mélange total de monomères. L'émulsion inverse finale eau-dans-huile contient, quant à elle, et de préférence, environ 40% en poids de copolymère réticulé tel que défini ci-avant et de l'ordre de 4% en poids d'un alcool gras éthoxylé ayant un HLB d'environ 12,5.

Les émulsions acides de type huile-dans-eau conformes à l'invention contiennent généralement des copolymères réticulés tels que ci-dessus définis en une quantité pondérale pouvant aller de 0,01 à 5% ramenée au poids de l'ensemble de l'émulsion, de préférence comprise entre 0,1 et 3%, toujours ramenée au poids de l'ensemble de l'émulsion. On notera que le copolymère réticulé servant d'agent de stabilisation de l'émulsion est essentiellement présent dans la phase aqueuse de cette dernière. En outre, selon la quantité de copolymère réticulé présente dans l'émulsion, on peut conférer à cette dernière des fluidités diverses et variées allant notamment de celle correspondant à un lait à celle correspondant à une crème. Ainsi, à l'inverse des produits utilisés dans les techniques antérieures (stabilisation d'émulsions H/E non, ou faiblement, acides par simple gélification mécanique de ces dernières), le copolymère réticulé utilisé dans le cadre de la présente invention exerce, sur les émulsions H/E de caractère fortement acide, une fonction générale de stabilisation utilisé à plus fortes concentrations, ledit copolymère exerce alors une double fonction, à savoir à la fois une fonction de stabilisation et une fonction d'épaississement des émulsions H/E fortement acides.

Dans les émulsions selon l'invention, la phase huileuse représente avantageusement de 2 à 35% en poids de l'ensemble de l'émulsion. Encore plus préférentiellement, cette proportion est de 5 à 30% en poids.

Comme souligné précédemment, cette phase grasse peut être constituée par tous les composés qui sont déjà connus de façon générale comme convenant pour la fabrication d'émulsions de type huile dans eau. En particulier, ces composés peuvent être choisis, seuls ou en mélanges, parmi les différents corps gras, les huiles d'origine végétale, animale ou minérale, les cires naturelles ou synthétiques, et analogues.

Parmi les huiles pouvant rentrer dans la composition de la phase grasse, on peut notamment citer:
- les huiles minérales telles que l'huile de paraffine et l'huile de vaseline,
- les huiles d'origine animale telles que le perhydrosqualène,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore et l'huile de seigle,
- les huiles synthétiques telles que l'huile de purcellin, le myristate de butyle, le myristate d'isopropyle, le myristate de cétyle, le palmitate d'isopropyle, le stéarate de butyle, le stéarate d'hexadécyle, le stéarate d'isopropyle, le stéarate d'octyle, le stéarate d'isocétyle, I'oléate de décyle, le laurate d'hexyle, le dicaprylate de propylène glycol et les esters dérivés d'acide lanolique tels que le lanolate d'isopropyle, le lanolate d'isocétyle et les isoparaffines.

Comme autres huiles synthétiques utilisables dans les émulsions selon l'invention, on peut encore citer les acétylglycérides, les octanoates et décanoates d'alcools et de polyalcools tels que ceux de glycol et de glycérol, les ricinoléates d'alcools et de polyalcools tels que ceux de cétyle, les triglycérides d'acides gras tels que les triglycérides caprylique/caprique, les triglycérides d'acides gras saturés en C₁₀-C₁₈, les huiles fluorées et perfluorées, et enfin les huiles silicones, volatiles ou non.

Bien entendu, la phase grasse peut également contenir un ou plusieurs actifs cosmétiques et/ou thérapeutiques lipophiles, notamment ceux qui sont déjà utilisés de manière habituelle dans la fabrication et l'obtention des compositions cosmétiques et/ou pharmaceutiques Ces actifs peuvent être, par exemple, des agents antiradicaux libres, des céramides, des filtres solaires tels que le paraméthoxycinnamate de 2-éthylhexyle et en particulier celui commercialisé sous la dénomination "Parsol MCX" par la Société GIVAUDAN ou la 2-hydroxy-4-méthoxybenzophénone et en particulier celle vendue sous la dénomination commerciale "Uvinul M40" par la Société BASF, des répulsifs pour insectes tels que le cétyl-3 éthylaminopropionate de n-butyle ou le diéthylamide-N,N-caprylique, des agents amincissants tels que le nicotinate de D-L α-tocophérol, l'extrait huileux de ginseng (Panax Ginseng), l'extrait huileux de lierre grimpant (Hedera Helix), l'extrait huileux de fleurs sèches d'arnica (Arnica montana L) et l'extrait huileux d'algues (Fucus Vesiculosus). Il va de soi que la liste ci-dessus d'actifs lipophiles susceptibles de rentrer dans la composition de la phase grasse n'est nullement exhaustive.

La phase grasse peut également contenir, selon l'application envisagée, un ou plusieurs additifs de formulation lipophiles tels que des agents conservateurs, des agents antioxydants, des agents émollients ou des huiles parfumées.

La taille des particules de phase grasse au sein de la phase aqueuse dispersante peut varier dans de larges limites, et elle est choisie en particulier en fonction de l'application finale recherchée; elle peut ainsi être soit submicronique (émulsoïdes), soit aller de 1 à plusieurs microns par exemple.

Dans les émulsions selon l'invention, la phase aqueuse représente avantageusement de 65 à 98% en poids de l'ensemble de l'émulsion. Encore plus préférentiellement, cette proportion est comprise entre 70 et 95% en poids.

De manière classique, cette phase aqueuse peut être constituée par de l'eau ou bien encore un mélange d'eau et d'alcools inférieurs hydrosolubles (C₁-C₆), et elle peut en outre contenir des actifs cosmétiques et/ou thérapeutiques, également hydrosolubles.

Parmi les adjuvants susceptibles d'être contenus dans la phase aqueuse, on peut citer notamment:
- des dérivés hydrosolubles tels que des colorants et des conservateurs,
- des principes actifs tels que l'acide hyaluronique et ses dérivés, ou le gluconate de magnésium,
- des oligo-éléments,
- des dérivés biologiques tels que l'urée, l'acide pyrrolidone carboxylique et ses différents sels notamment son sel de sodium,
- des polyols tels que le propylène glycol, le butylène 1,3- glycol, le glycérol, le polyglycérol, le sorbitol, le glucose et le saccharose,
- des sels tels que le sulfate de magnésium et le chlorure de sodium,
- des minéraux argileux gonflant en milieu aqueux tels que la saponite, l'hectorite et la smectite,
- des acides aminés tels que la proline et l'hydroxyproline,
- des protéines telles que les kératines sulfoniques, le collagène, I'élastine et l'ADN,
- des agents adoucissants,
- des agents antibactériens tels que le transthiolanediol 3,4 S-dioxyde,
- des filtres solaires hydrosolubles tels que l'acide 2-hydroxy 4-méthoxy benzophénone 5-sulfonique, par exemple celui commercialisé sous la dénomination "Uvinul MS40" par la Société BASF,
- des agents amincissants tels que la caféine, la théobromine, la théophylline, la L-carnitine, le chlorhydrate de diméthylaminoéthylthéophylline,
- les dérivés du silicium du type méthylsilanol théophyllinacétate alginate,
- des dérivés végétaux tels que les extraits hydroglycoliques de lierre grimpant, d'algue brune, de pensée sauvage fraîche,
- des produits pour jambes lourdes tels que le ginkgo biloba, le mélilot ou le ruscus.

Les émulsions selon l'invention peuvent en outre contenir, généralement à raison de 0,3 à 20% en poids par rapport à l'ensemble de l'émulsion, de préférence à raison de 0,5 à 10 % en poids, des tensio-actifs ou émulsionnants dont l'emploi a pu être rendu nécessaire lors de la préparation de l'émulsion initiale non encore stabilisée conformément à l'invention.

De tels tensio-actifs sont tous les tensio-actifs non-ioniques connus de l'homme de l'art pour réaliser de manière classique une émulsion de type huile dans eau. A titre d'exemples de tensio-actifs non ioniques convenables, on peut citer notamment:
- les esters d'acides gras du sorbitan, oxyéthylénés ou non, tels que le laurate, le palmitate, le stéarate, I'oléate ou le tristéarate de sorbitan, en particulier ceux vendus sous la dénomination de "Span" par la Société ICI, et les composés oxyéthylénés tels que les polysorbates commercialisés sous la dénomination de "Tween" par la société ICI,
- les esters d'acides gras de polyéthylèneglycols tels que le PEG-8 stéarate, le PEG-20 stéarate, le PEG-30 stéarate, le PEG-40 stéarate, le PEG-50 stéarate et le PEG-100 stéarate, en particulier ceux vendus sous la dénomination de "Myrj" par la Société ICI,
- les esters d'acides gras de glycérol tels que le stéarate ou l'oléate de glycéryl ou les mélanges les contenant, en particulier ceux vendus sous les noms de "Tegin" par la Société GOLDSCHMIDT et de "Arlacel 165" et "Arlacel 186" par la Société ICI,
- les polyéthylèneglycol éthers d'esters d'acides gras de glycéryl, tels que les PEG-Glycéryl stéarate ou oléate ou laurate, par exemple les produits vendus sous le nom de "Arlatone 983S" par la Société ICI,
- les alcools gras polyéthoxylés, polypropoxylés ou polyglycérolés à chaine grasse comportant de 8 à 18 atomes de carbone,
- les acides gras polypropoxylés et polyglycérolés,
- les copolymères d'oxydes d'éthylène et de propylène,
- les amines ou amides gras polyéthoxylés tels que les PEG-cocoamide, PEG-cocamine, les PEG-lauramide, PEG-oléamide, PEG-oléamine, PEG-stéaramide et PEG-stéaramine,
- les esters et éthers gras de sucres, en particulier de glucose et de sucrose, tels que les produits vendus sous les dénominations de "Grilloten" par la Société RITA et de "Grillocose" par la Société GRILLOWERKE et de "Glucate et Glucamate" par la Société AMERCHOL,
- les alkylpolyglycosides et les mélanges les contenant tels que les produits vendus sous les dénominations "Montanov 68" et "Oramix" par la Société SEPPIC.

D'autres agents tensio-actifs susceptibles d'être présents dans les émulsions selon l'invention sont :
- les produits de condensation d'un monoalcool, d'un α-diol, d'un alcoylphénol, d'un amide ou d'un diglycolamide avec le glycidol ou un précurseur de glycidol, en particulier ceux répondant à la formule ci-dessous : 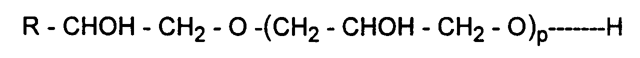 dans laquelle R désigne un radical aliphatique, cycloaliphatique ou arylaliphatique ayant de préférence entre 7 et 21 atomes de carbone, les chaines aliphatiques pouvant comporter des groupements éthers, thioéthers ou hydroxyméthylène, et où p est une valeur statistique comprise entre 1 et 10 inclus, tels que décrits dans la demande de brevet français FR-A- 2091 516,
- les composés répondant à la formule : 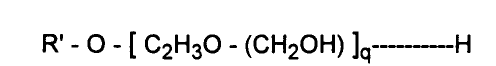 dans laquelle R' désigne un radical alcoyle, alcényle ou alcoylaryle en C₈-C₃₀, et q est une valeur statistique comprise entre 1 et 10 inclus, tels que décrits dans la demande de brevet français FR-A- 1 477 048,
- les composés répondant à la formule : 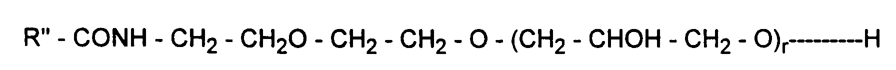 dans laquelle R'' désigne un radical aliphatique linéaire ou ramifié, saturé ou insaturé, comportant éventuellement un ou plusieurs groupements hydroxyle, et ayant entre 8 et 30 atomes de carbone, et r représente un nombre entier ou décimal compris entre 1 et 5 (degré de condensation moyen), tels que décrits dans la demande de brevet français FR-A- 2 328 763.

Il est également possible, et ceci de manière bien connue, que les émulsions selon l'invention contiennent des co-émulsionnants, dont le rôle est, lors de la préparation de l'émulsion initiale (non encore stabilisée), de diminuer de manière substantielle la quantité d'agents tensio-actifs nécéssaire à la réalisation de ladite émulsion initiale. Dans cette famille de produits, on peut ainsi citer les alcools gras ou les acides gras en C₁₄-C₁₈, les alkyléthers de glycéryle dont la chaine alkyle est en C₁₄-C₂₂, ainsi que les composés de formule : 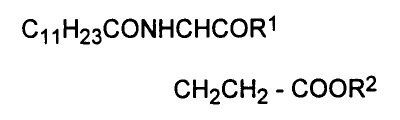 dans laquelle R¹ et R², qui peuvent être identiques ou différents, représentent un radical cholestéryle, béhényle ou 2-octyldodécyle.

A titre d'exemples particuliers d'agents co-émulsionnants, on peut notamment citer l'alcool stéarylique, l'alcool cétylique, l'acide stéarique, l'alcool chimylique, l'alcool bétylique, l"Eldew CL-301" commercialisé par la Société AJINOMOTO, et la cire de Sumac qui contient un mélange d'acides gras.

Comme indiqué en début de description, l'un des intérêts majeurs des émulsions de type huile-dans-eau conformes à l'invention est que ces dernières peuvent contenir, tout en présentant un caractère stable, des principes actifs, tant cosmétiques que thérapeutiques, à caractère fortement acide, ces actifs pouvant donc être notamment choisis parmi tous ceux habituellement utilisés à ce jour dans le domaine de la cosmétique, de la dermatologie ou du médicament.

On peut ainsi citer, entre autres, l'acide ascorbique, l'acide kojique, l'acide caféique, l'acide salicylique et ses dérivés, les alpha-hydroxyacides tels que l'acide lactique, l'acide méthyllactique, l'acide mandélique, l'acide glucuronique, l'acide glycolique, l'acide pyruvique, l'acide 2-hydroxybutanoïque, l'acide 2-hydroxypentanoïque, l'acide 2-hydroxyhexanoïque, l'acide 2-hydroxyheptanoïque, l'acide 2-hydroxyoctanoïque, l'acide 2-hydroxynonanoïque, l'acide 2-hydroxydécanoïque, l'acide 2-hydroxyundécanoïque, l'acide 2-hydroxydodécanoïque, l'acide 2-hydroxytetradécanoïque, l'acide 2-hydroxyhexadecanoïque, l'acide 2-hydroxyoctadécanoïque, l'acide 2-hydroxytetraéicosanoïque, l'acide 2-hydroxyeicosanoïque, l'acide benzylique, l'acide phényllactique, l'acide gluconique, l'acide galacturonique, l'acide citrique, l'acide aleuritique, l'acide ribonique, l'acide tartronique, l'acide tartrique, l'acide malique, l'acide fumarique, l'acide rétinoïque et ses dérivés, l'acide benzène 1,4-di(3-méthylidène 10-camphosulfonique). Il peut s'agir également de tous les composés naturels ou synthétiques contenant de tels acides, en particulier les extraits végétaux, et plus spécialement les extraits de fruits.

Comme indiqué précédemment, les compositions à usage topique selon l'invention, sous forme essentiellement d'émulsions acides de type H/E, peuvent être utilisées dans différentes applications cosmétiques et/ou dermatologiques et/ou thérapeutiques, par exemple sous forme de crèmes pour le visage, pour le corps, pour le cuir chevelu ou pour la chevelure, ou bien encore sous forme de laits pour le corps ou pour le démaquillage. Elles peuvent être également utilisées pour le maquillage, notamment sous forme de fonds de teint, après addition de pigments dans ces dernières. Elles peuvent être aussi utilisées comme crèmes solaires après addition de filtres UVB et/ou UVA, ou comme crèmes ou laits après solaire après addition de composés apaisants tels que le panthènol.

Les émulsions acides de type huile-dans-eau destinées à être stabilisées selon l'invention peuvent avoir été obtenues selon toute technique connue en soi pour la préparation de ce type de compositions, par exemple en introduisant, sous agitation énergique (la puissance de l'agitation permettant en particulier de régler la taille des particules constituant la phase grasse dispersée) et le plus souvent en présence d'agents émulsionnants, la phase grasse (éventuellement préalablement chauffée pour augmenter sa fluidité) dans une phase aqueuse maintenue à une température généralement comprise entre 70 et 80°C, les éventuels additifs, actifs ou non, hydrophiles ou lipophiles pouvant être soit initialement présents dans les phases aqueuse, respectivement huileuse, à mélanger, soit amenés séparément lors de la synthèse même de l'émulsion, ou après cette synthèse. On notera que, dans le cadre de la présente invention, le mode de préparation de l'émulsion initiale ne revêt en fait absolument aucun caractère critique.

Le principe de la stabilisation de l'émulsion telle ci-dessus préparée consiste alors simplement à introduire dans cette dernière, dans une étape ultime, l'agent de stabilisation conforme à l'invention.

Des exemple concrets de compositions illustrant l'invention vont maintenant être donnés.

Le mode opératoire commun qui a été suivi pour préparer ces compositions était le suivant:

On a versé la phase grasse **(A)** dans la phase aqueuse **(B)** préalablement chauffée et maintenue à une température comprise entre 70 et 75°C, et ceci sous une agitation énergique conduite au moyen d'une turbine type MORITZ; lorsque la finesse désirée pour l'émulsion était atteinte, on a refroidi le milieu, sous agitation aux pâles, jusqu'à 35-40°C; lorsque cette dernière température était atteinte, on a ajouté dans l'émulsion des actifs acides **(C)**, sous agitation turbine; et enfin on a stabilisé l'émulsion ainsi obtenue en y introduisant un agent stabilisant conforme à l'invention **(D)** et qui, pour tous les exemples, correspondait au produit tel que préparé à l'exemple 1 de la demande de brevet EP-A-0 503 853 déjà citée, c'est à dire un produit se présentant sous la forme d'une émulsion de type eau dans huile et contenant 40% en poids environ d'un copolymère réticulé acrylamide (60% en mole) / AMPS sodé (40% en mole) / Méthylène bis-acrylamide (0,22 millimole/mole), et 4% en poids environ d'un alcool gras éthoxylé ayant un HLB d'environ 12,5.

Dans tous les exemples qui suivent, les compositions sont définies en % en poids.

### Exemple 1

Cette exemple illustre la préparation et l'obtention d'un lait corporel hydratant de pH 2,8 à base d'une émulsion H/E conforme à l'invention.

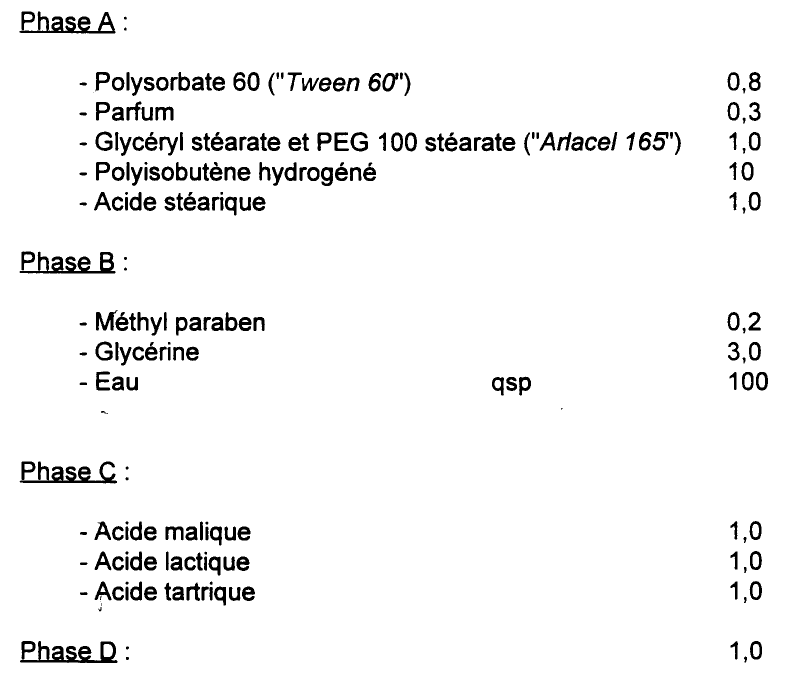

La composition obtenue est parfaitement stable.

### Exemple 2

Cet exemple illustre la préparation et l'obtention d'une crème de soin de pH 2,2 à base d'une émulsion H/E conforme à l'invention.

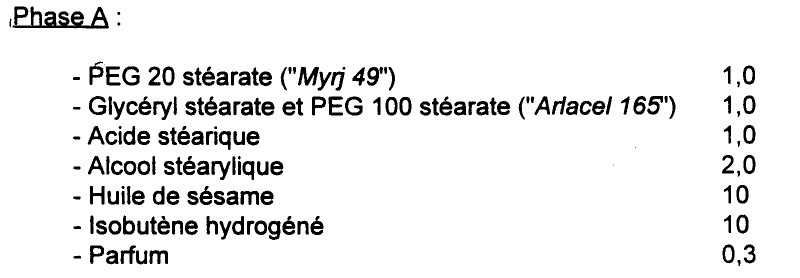

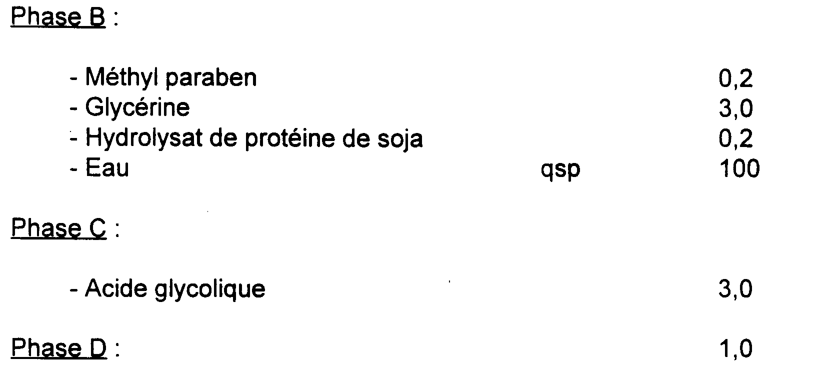

La composition obtenue est parfaitement stable.

### Exemple 3

Cet exemple illustre la préparation et l'obtention d'un fluide de soin pour peaux grasses, de pH 2,8 et à base d'une émulsion H/E conforme à l'invention.

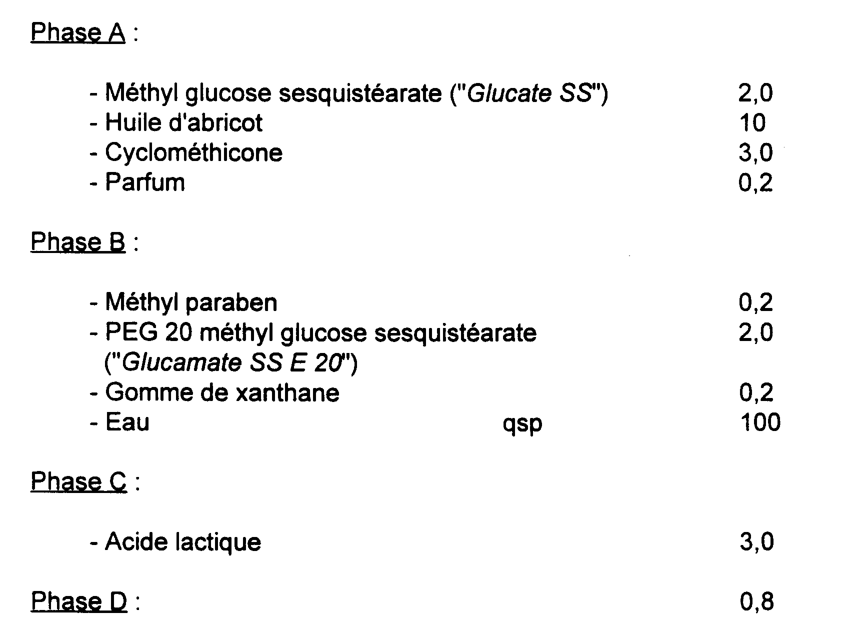

La composition obtenue est parfaitement stable.

## Revendications

1. Une émulsion de type huile-dans-eau, stable et de pH inférieur ou égal 3,5, contenant un ou plusieurs agents acides cosmétiquement et/ou thérapeutiquement actifs et comprenant, à titre d'agent stabilisant, un copolymère anionique réticulé substantiellement soluble dans l'eau et constitué de motifs dérivant de la réaction entre (i) l'acrylamide, (ii) l'acide 2-acrylamido 2-méthyl propane sulfonique (AMPS) et (iii) au moins un composé à polyinsaturation oléfinique (agent de réticulation), ledit composé à polyinsaturation oléfinique étant présent dans le copolymère à une concentration comprise entre 0,06 et 1 millimole par mole de l'ensemble des motifs monomères.

2. Emulsion selon la revendication 1, caractérisée en ce qu'elle présente un pH inférieur ou égal à 3.

3. Emulsion selon l'une des revendications 1 ou 2, caractérisée en ce que, dans ledit copolymère, le rapport, exprimé en mole %, entre les motifs acrylamide et AMPS est compris entre 85/15 et 15/85.

4. Emulsion selon la revendication 3, caractérisée en ce que ledit rapport est compris entre 70/30 et 30/70.

5. Emulsion selon la revendication 4, caractérisée en ce que ledit rapport est compris entre 65/35 et 35/65.

6. Emulsion selon la revendication 5, caractérisée en ce que ledit rapport est compris entre 60/40 et 40/60.

7. Emulsion selon l'une quelconque des revendications précédentes, caractérisée en ce que l'AMPS est, pour partie au moins, neutralisé sous la forme d'un sel.

8. Emulsion selon l'une quelconque des revendications précédentes, caractérisée en ce que ledit composé à polyinsaturation oléfinique est choisi parmi le méthylène bis-acrylamide, l'allyle-sucrose et le pentaérythritol.

9. Emulsion selon la revendication 8, caractérisée en ce que ledit composé à polyinsaturation oléfinique est le méthylène bis-acrylamide.

10. Emulsion selon l'une quelconque des revendications précédentes, caractérisée en ce que ledit copolymère est présent à raison de 0,01 à 5% en poids par rapport à l'ensemble de l'émulsion.

11. Emulsion selon la revendication 10, caractérisée en ce que la quantité de copolymère est comprise entre 0,1 et 3% en poids.

12. Emulsion selon l'une quelconque des revendications précédentes, caractérisée en ce que la phase huileuse est constituée de corps gras, d'huiles végétales, animales ou minérales, et/ou de cires naturelles ou synthétiques.

13. Emulsion selon l'une quelconque des revendications précédentes, caractérisée en ce que la phase huileuse représente de 2 à 35% en poids de l'ensemble de l'émulsion.

14. Emulsion selon la revendication 13, caractérisée en ce que la quantité de phase huileuse est comprise entre 5 et 30% en poids.

15. Emulsion selon l'une quelconque des revendications précédentes, caractérisée en ce que la phase aqueuse est constituée d'eau ou d'un mélange d'eau et d'alcools inférieurs hydrosolubles.

16. Emulsion selon l'une quelconque des revendications précédentes, caractérisée en ce que la phase aqueuse représente de 65 à 98% en poids de l'ensemble de l'émulsion.

17. Emulsion selon la revendication 16, caractérisée en ce que la quantité de phase aqueuse est comprise entre 70 et 95%.

18. Emulsion selon l'une quelconque des revendications précédentes, caractérisée en ce qu'elle comprend des agents émulsionnants.

19. Emulsion selon la revendication 18, caractérisée en ce que lesdits agents émulsionnants sont présents à raison de 0,3 à 20% en poids par rapport à l'ensemble de l'émulsion.

20. Emulsion selon la revendication 19, caractérisée en ce que la quantité d'agents émulsionnants est comprise entre 0,5 et 10%.

21. Emulsion selon l'une quelconque des revendications précédentes, caractérisée par le fait qu'elle constitue une composition pour usage topique.

22. Utilisation d'un copolymère réticulé tel que défini à l'une quelconque des revendications 1 et 3 à 9, pour la stabilisation d'une émulsion acide de type huile-dans-eau de pH inférieur ou égal à 3,5, en particulier inférieur ou égal à 3.

23. Utilisation selon la revendication 22, caractérisée en ce que ledit copolymère réticulé se présente sous la forme d'une émulsion inverse de type eau-dans-huile.

24. Utilisation selon la revendication 23, caractérisée en ce que ladite émulsion inverse contenant ledit copolymère réticulé comprend un alcool gras éthoxylé ayant un HLB d'environ 12,5.

## Claims

1. An oil-in-water type emulsion which is stable and has a pH below or equal to 3.5, containing one or more cosmetically and/or therapeutically active acidic agents and comprising as stabilizing agent a substantially water-soluble crosslinked anionic copolymer consisting of units derived from the reaction between (i) acrylamide, (ii) 2-acrylamido-2-methylpropanesulphonic acid (AMPS) and (iii) at least one compound possessing multiple olefinic unsaturation (crosslinking agent), the said compound possessing multiple olefinic unsaturation being present in the copolymer at a concentration of between 0.06 and 1 mmol per mole of the collective monomer units.

2. Emulsion according to Claim 1, characterized in that it possesses a pH below or equal to 3.

3. Emulsion according to either of Claims 1 and 2, characterized in that, in the said copolymer, the ratio, expressed in mol %, of acrylamide to AMPS units is between 85:15 and 15:85.

4. Emulsion according to Claim 3, characterized in that the said ratio is between 70:30 and 30:70.

5. Emulsion according to Claim 4, characterized in that the said ratio is between 65:35 and 35:65.

6. Emulsion according to Claim 5, characterized in that the said ratio is between 60:40 and 40:60.

7. Emulsion according to any one of the preceding claims, characterized in that the AMPS is at least partially neutralized in the form of a salt.

8. Emulsion according to any one of the preceding claims, characterized in that the said compound possessing multiple olefinic unsaturation is chosen from methylenebisacrylamide, allylsucrose and pentaerythritol.

9. Emulsion according to Claim 8, characterized in that the said compound possessing multiple olefinic unsaturation is methylenebis- acrylamide.

10. Emulsion according to any one of the preceding claims, characterized in that the said copolymer is present in the proportion of 0.01 to 5 % by weight relative to the whole of the emulsion.

11. Emulsion according to Claim 10, characterized in that the amount of copolymer is between 0.1 and 3 % by weight.

12. Emulsion according to any one of the preceding claims, characterized in that the oily phase consists of fats, vegetable, animal or mineral oils and/or natural or synthetic waxes.

13. Emulsion according to any one of the preceding claims, characterized in that the oily phase represents from 2 to 35 % by weight of the whole of the emulsion.

14. Emulsion according to Claim 13, characterized in that the amount of oily phase is between 5 and 30 % by weight.

15. Emulsion according to any one of the preceding claims, characterized in that the aqueous phase consists of water or of a mixture of water and water-soluble lower alcohols.

16. Emulsion according to any one of the preceding claims, characterized in that the aqueous phase represents from 65 to 98 % by weight of the whole of the emulsion.

17. Emulsion according to Claim 16, characterized in that the amount of aqueous phase is between 70 and 95 %.

18. Emulsion according to any one of the preceding claims, characterized in that it comprises emulsifying agents.

19. Emulsion according to Claim 18, characterized in that the said emulsifying agents are present in the proportion of 0.3 to 20 % by weight relative to the whole of the emulsion.

20. Emulsion according to Claim 19, characterized in that the amount of emulsifying agents is between 0.5 and 10 %.

21. Emulsion according to any one of the preceding claims, characterized in that it constitutes a composition for topical use.

22. Use of a crosslinked copolymer as defined in any one of Claims 1 and 3 to 9, for the stabilization of an acidic oil-in-water type emulsion having a pH below or equal to 3.5, especially below or equal to 3.

23. Use according to Claim 22, characterized in that the said crosslinked copolymer takes the form of a water-in-oil type reverse emulsion.

24. Use according to Claim 23, characterized in that the said reverse emulsion containing the said crosslinked copolymer comprises an ethoxylated fatty alcohol having an HLB of approximately 12.5.

## Patentansprüche

1. Emulsion vom Öl-in-Wasser-Typ, die stabil ist, die einen pH-Wert von 3,5 oder darunter aufweist, die einen oder mehrere kosmetische und/oder therapeutische Wirkstoffe enthält und die als Stabilisierungsmittel ein vernetztes, anionisches Copolymer aufweist, das im wesentlichen wasserlöslich ist und das aus Einheiten besteht, die aus der Umsetzung von (i) Acrylamid, (ii) 2-Acrylamido-2-methylpropansulfonsäure (AMPS) und (iii) mindestens einer Verbindung mit olefinischer Doppelbindung (Vernetzungsmittel) stammen, wobei die Verbindung mit olefinischer Doppelbindung in dem Copolymer in einer Konzentration im Bereich von 0,06 bis 1 mmol pro Mol der gesamten Monomereinheiten vorliegt.

2. Emulsion nach Anspruch 1, dadurch gekennzeichnet, daß sie einen pH-Wert von 3 oder darunter aufweist.

3. Emulsion nach einem der Ansprüche 1 oder 2, dadurch gekennzeichnet, daß das in Mol-% ausgedrückte Verhältnis der Acrylamideinheiten und AMPS-Einheiten in dem Copolymer im Bereich von 85/15 bis 15/85 liegt.

4. Emulsion nach Anspruch 3, dadurch gekennzeichnet, daß das Verhältnis im Bereich von 70/30 bis 30/70 liegt.

5. Emulsion nach Anspruch 4, dadurch gekennzeichnet, daß das Verhältnis im Bereich von 65/35 bis 35/65 liegt.

6. Emulsion nach Anspruch 5, dadurch gekennzeichnet, daß das Verhältnis im Bereich von 60/40 bis 40/60 liegt.

7. Emulsion nach einem der vorhergehenden Ansprüche, dadurch gekennzeichnet, daß die AMPS zumindest teilweise neutralisiert in Form eines Salzes vorliegt.

8. Emulsion nach einem der vorhergehenden Ansprüche, dadurch gekennzeichnet, daß die Verbindung mit olefinischer Doppelbindung unter Methylen-bis-acrylamid, Allyl-saccharose oder Pentaerythrit ausgewählt ist.

9. Emulsion nach Anspruch 8, dadurch gekennzeichnet, daß die Verbindung mit olefinischer Doppelbindung das Methylen-bis-acrylamid ist.

10. Emulsion nach einem der vorhergehenden Ansprüche, dadurch gekennzeichnet, daß das Copolymer in einem Anteil von 0,01 bis 5 Gew.-%, bezogen auf die gesamte Emulsion, vorliegt.

11. Emulsion nach Anspruch 10, dadurch gekennzeichnet, daß der Mengenanteil des Copolymers im Bereich von 0,1 bis 3 Gew.-% liegt.

12. Emulsion nach einem der vorhergehenden Ansprüche, dadurch gekennzeichnet, daß die Ölphase aus Fettsubstanzen, pflanzlichen, tierischen oder mineralischen Ölen und/oder natürlichen oder synthetischen Wachsen besteht.

13. Emulsion nach einem der vorhergehenden Ansprüche, dadurch gekennzeichnet, daß die Ölphase 2 bis 35 Gew.-% der gesamten Emulsion ausmacht.

14. Emulsion nach Anspruch 13, dadurch gekennzeichnet, daß der Mengenanteil der Ölphase im Bereich von 5 bis 30 Gew.-% liegt.

15. Emulsion nach einem der vorhergehenden Ansprüche, dadurch gekennzeichnet, daß die wäßrige Phase aus Wasser oder einem Gemisch von Wasser und wasserlöslichen, niederen Alkoholen besteht.

16. Emulsion nach einem der vorhergehenden Ansprüche, dadurch gekennzeichnet, daß die wäßrige Phase 65 bis 98 Gew.-% der gesamten Emulsion ausmacht.

17. Emulsion nach Anspruch 16, dadurch gekennzeichnet, daß der Mengenanteil der wäßrigen Phase im Bereich von 70 bis 95 Gew.-% liegt.

18. Emulsion nach einem der vorhergehenden Ansprüche, dadurch gekennzeichnet, daß sie Emulgatoren enthält.

19. Emulsion nach Anspruch 18, dadurch gekennzeichnet, daß die Emulgatoren in einem Mengenanteil von 0,3 bis 20 Gew.-%, bezogen auf die gesamte Emulsion, vorliegen.

20. Emulsion nach Anspruch 19, dadurch gekennzeichnet, daß der Mengenanteil der Emulgatoren im Bereich von 0,5 bis 10 Gew.-% liegt.

21. Emulsion nach einem der vorhergehenden Ansprüche, dadurch gekennzeichnet, daß sie eine Zusammensetzung zur topischen Anwendung darstellt.

22. Verwendung eines vernetzten Copolymers nach einem der Ansprüche 1 und 3 bis 9 zur Stabilisierung einer sauren Emulsion vom Öl-in-Wasser-Typ mit einem pH-Wert von 3,5 oder darunter und insbesondere 3 oder darunter.

23. Verwendung nach Anspruch 22, dadurch gekennzeichnet, daß das vernetzte Copolymer in Form einer inversen Emulsion vom Wasser-in-Öl-Typ vorliegt.

24. Verwendung nach Anspruch 23, dadurch gekennzeichnet, daß die inverse Emulsion, die das vernetzte Copolymer enthält, einen ethoxylierten Fettalkohol mit einem HLB-Wert von etwa 12,5 enthält.
